(19) Europäisches Patentamt
European Patent Office
Office européen des brevets

(11) EP 1 110 944 A2

(12) **EUROPÄISCHE PATENTANMELDUNG**

(43) Veröffentlichungstag:
**27.06.2001 Patentblatt 2001/26**

(51) Int Cl.[7]: **C07C 231/02**, C07C 233/18, C07C 233/20

(21) Anmeldenummer: **00127022.2**

(22) Anmeldetag: **09.12.2000**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **24.12.1999 DE 19962999**

(71) Anmelder: **Clariant GmbH**
**65929 Frankfurt am Main (DE)**

(72) Erfinder: **Weinelt, Frank, Dr.**
**84508 Burgkirchen (DE)**

(54) **Verfahren zur Herstellung von Festtsäure-N-Alkylpolyhydroxyamiden**

(57) Verfahren zur Herstellung von Fettsäure-N-alkylpolyhydroxyamiden der Formel

$$R^1CONR^2Z$$

worin $R^1$ für eine lineare oder verzweigte, gesättigte oder ungesättigte Alkylgruppe mit 7 bis 21 Kohlenstoffatomen, Z für eine Polyhydroxykohlenwasserstoffgruppe mit mindestens drei Hydroxylgruppen, die auch alkoxyliert sein können, und $R^2$ für H, $C_1$-$C_8$-Alkyl, eine Gruppe der Formeln $-(CH_2)_xNR^3R^4$ oder $R^5O(CH_2)_n$-, $R^3$ und $R^4$ für $C_1$-$C_4$-Alkyl oder $C_2$-$C_4$-Hydroxyalkyl, $R^5$ für $C_1$-$C_4$-Alkyl, n für eine Zahl von 2 bis 4 und x für eine Zahl von 2 bis 10, stehen, durch Umsetzung eines N-Alkyl-polyhydroxyamins der Formel

$$H\text{-}NR^2Z$$

mit einer Fettsäure, Fettsäurechlorid oder Fettsäureesters in Gegenwart eines basischen Katalysators, dadurch gekennzeichnet, dass man die Reaktion in Gegenwart eines Fettsäureamidoalkoxylats der Formel

$$R\text{-}CONR^6R^7$$

durchführt, wobei R Alkyl oder Alkenyl mit 7 bis 21 Kohlenstoffatomen, $R^6$ Wasserstoff oder eine Gruppe $-(AO)_xH$, $R^7$ eine Gruppe $-(AO)_xH$, A eine Gruppe der Formeln $-C_2H_4-$, $-C_3H_6-$ oder $-C_4H_8-$ und x eine Zahl von 1 bis 20 bedeuten.

EP 1 110 944 A2

## Beschreibung

Die Erfindung betrifft ein verbessertes Verfahren zur Herstellung von Fettsäure-N-Alkylpolyhydroxyamiden der Formel I

$$R^1CONR^2Z \qquad (I)$$

worin $R^1$ für eine lineare oder verzweigte, gesättigte oder ungesättigte Alkylgruppe mit 7 bis 21 Kohlenstoffatomen, Z für eine Polyhydroxykohlenwasserstoffgruppe mit mindestens drei Hydroxylgruppen, die auch alkoxyliert sein können, und $R^2$ für H, $C_1$-$C_8$-Alkyl, eine Gruppe der Formeln $-(CH_2)_xNR^3R^4$ oder $R^5O(CH_2)_n-$, $R^3$ und $R^4$ für $C_1$-$C_4$-Alkyl oder C2-C4-Hydroxyalkyl, $R^5$ für $C_1$-$C_4$-Alkyl, n für eine Zahl von 2 bis 4 und x für eine Zahl von 2 bis10, stehen.
Fettsäure-N-Alkyl-polyhydroxyamide stellen nichtionische Tenside auf Basis nachwachsender Rohstoffe dar, die wegen ihrer ausgezeichneten anwendungstechnischen Eigenschaften und ihrer besonderen ökotoxikologischen Verträglichkeit zunehmend für die Herstellung oberflächenaktiver Mittel, aber auch für kosmetische Produkte, sowie in Pflanzenschutzmitteln etc. an Bedeutung gewinnen.

Die Verbindungen der Formel I werden, wie in WO 92/06160, sowie in der dort angegebenen Literatur beschrieben, durch reduktive Aminierung eines reduzierenden Zuckers mit Ammoniak, einem Alkylamin oder einem Alkanolamin zu einem N-Alkyl-Polyhydroxyamin der Formel II

$$H\text{-}NR^2Z \qquad (II)$$

wobei Z und $R^2$ die oben genannte Bedeutung haben, und anschließende Acylierung mit einer Fettsäure, Fettsäurechlorid oder Fettsäureester hergestellt.
Zur Acylierung der Amine der Formel II werden diese bei Temperaturen vorzugsweise von 125 bis 130°C aufgeschmolzen und mit dem Acylierungsmittel zur Reaktion gebracht. Nachteilig sind neben dem hohen Energieeintrag die Bildung unerwünschter Nebenprodukte, insbesondere cyclischer Zuckerderivate.

Es bestand daher die Aufgabe ein verbessertes Verfahren zur Herstellung dieser Tensidklasse zu entwickeln.

Überraschend wurde gefunden, dass die Synthese von Fettsäure-N-Alkylpolyhydroxyamiden der Formel I durch Acylierung der Amine gemäß der Formel II in Gegenwart eines basischen Katalysators in kürzeren Reaktionszeiten erfolgt, wenn dem N-Alkyl-Polyhydroxyamin der Formel II Fettsäureamidoalkoxylate der Formel III zugesetzt werden.

$$R\text{-}CON(R^6)(R^7) \qquad (III)$$

worin R Alkyl oder Alkenyl mit 7 bis 21 Kohlenstoffatomen, $R^6$ Wasserstoff oder eine Gruppe $-(AO)_xH$, $R^7$ eine Gruppe $-(AO)_xH$, A eine Gruppe der Formeln $-C_2H_4-$, $-C_3H_6-$oder $-C_4H_8-$ und x eine Zahl von 1 bis 20 bedeuten.

Der Zusatz von Fettsäureamidoalkoxylat bewirkt eine Verminderung der Reaktionszeiten. Fettalkoholethoxylate anstelle von Fettsäureamidoalkoxylate zeigen dieses Verhalten nicht.

Das erfindungsgemäße Verfahren zur Herstellung von Fettsäure-N-Alkyl-Polyhydroxyalkylamiden gemäß der Formel I stellt einen bedeutenden ökonomischen Vorteil dar und bewirkt darüber hinaus anwendungstechnische Vorteile, wie verbesserte Löslichkeit und günstigeres Viskositätsverhalten der Produktmischung aus Amid gemäß Formel I und Fettsäureamidoalkoxylat der Formel III.

Vorzugsweise hergestellte Fettsäure-N-Alkyl-Polyhydroxyalkylamide leiten sich von reduzierenden Zuckern mit 5 oder 6 Kohlenstoffatomen, insbesondere von der Glucose ab.

Die bevorzugt hergestellten Fettsäure-N-Alkyl-Polyhydroxyalkylamide stellen daher Fettsäure-N-Alkyl-Glucamide dar, wie sie durch die Formel IV wiedergegeben werden

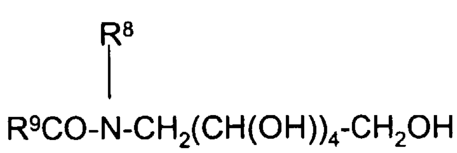

(IV)

Vorzugsweise werden als Fettsäure-N-Alkyl-Polyhydroxyalkylamide Glucamide der Formel IV hergestellt, in der $R^8$ für eine $C_1$-$C_4$-Alkylgruppe, insbesondere Methyl, und $R^9CO$ den Rest einer gesättigten oder ungesättigten Fettsäure mit 8 bis 22 C-Atomen bedeuten. Insbesondere bedeutet $R^9CO$ den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure, Talgfettsäure bzw. derer technischer Mischungen, besonders bevorzugt für $C_{12}$-$C_{18}$-, insbesondere $C_{12}$-$C_{14}$- und $C_{16}$-$C_{18}$-Fettsäuren.

Weiterhin können sich die mit dem erfindungsgemäßen Verfahren hergestellten Polyhydroxyalkylamide auch von Maltose und Palatinose ableiten.

Die im erfindungsgemäßen Verfahren zur Herstellung von Fettsäure-N-AlkylPolyhydroxyamiden eingesetzten Fettsäureamidoalkoxylate der Formel III werden durch Umsetzung von Fettsäuremethylester und Mono- oder Dialkanolamin und anschließender Alkoxylierung erhalten.

Die erfindungsgemäße Herstellung der Fettsäure-N-Alkyl-Polyhydroxyamide erfolgt vorzugsweise wie folgt: N-Alkyl-Polyhydroxyamin wird nach gängigen Verfahren in einer Strippapparatur im Temperaturbereich von 95 bis 135°C bei Normaldruck und anschließend bei reduziertem Druck im Bereich von 1000 mbar bis 20 mbar, bevorzugt 30 mbar bis zu einem Restwassergehalt von 0,01 bis 0,5 Gew.-%, bevorzugt 0,2 Gew.-% entwässert.
Dem bei einer Temperatur von 127 bis 130°C aufgeschmolzenen Amin wird auf 80 bis 100°C erwärmtes Fettsäureamidoalkoxylat in den Gewichtsmengen 10 bis 70 Gew.-%, bevorzugt 15 bis 40 Gew.-%, besonders bevorzugt 20 bis 30 Gew.-% bezogen auf die Gesamtreaktionsmischung und das Acylierungsmittel Fettsäure oder Fettsäurechlorid, insbesondere Fettsäurealkylester, in den Molmengen 100 bis 120 Mol-%, bevorzugt 105 bis 115 Mol-%, besonders bevorzugt 105 bis 110 Mol-%, bezogen auf den Aminanteil unter Rühren zugegeben und die Temperatur der Mischung oberhalb des Verfestigungspunktes belassen. Die Schmelztemperatur der Mischung ist im Vergleich zur Schmelztemperatur des Amins um 5 bis 7°C geringer.
Anschließend wird 2 bis 15 Mol-%, bevorzugt 5 bis 10 Mol-%, bezogen auf den Aminanteil, eines basischen Katalysators beispielsweise ein Metallalkoxyd, insbesondere Natriummethylat oder ein Alkalisalz, insbesondere das Natriumsalz von Glycerin oder Propylenglykol, bevorzugt gelöst in einem organischen Lösungsmittel, bevorzugt Methanol, oder ein Alkalihydroxyd, insbesondere NaOH im Zeitraum von 5 Minuten bis 10 Minuten zugetropft.

Innerhalb von 5 Minuten bis 10 Minuten startet die Reaktion unter Alkoholbildung (bzw. unter Bildung von $H_2O$ oder HCl), der bei 120°C bis 100°C bei Normaldruck, anschließend bei 100°C bis 80°C, bevorzugt bei 90°C bis 85°C im Vakuum bei 400 mbar bis 20 mbar, bevorzugt 100 mbar bis 20 mbar abdestilliert wird. Gleichzeitig mit der Alkoholbildung ist ein Klarwerden der Reaktionsmischung zu beobachten.

Die im erfindungsgemäßen Verfahren zur Herstellung von Fettsäure-N-AlkylPolyhydroxyamiden zugesetzten Fettsäureamidoalkoxylate bewirken eine Verkürzung der Anlaufphase bis zur Umsetzung von Amin mit Fettsäurealkylester in Gegenwart eines basischen Katalysators unter Alkoholbildung. Ersetzt man Fettsäureamidoalkoxylate durch Fettalkoholethoxylate oder Glykole, beispielsweise Propylenglykol, ist der Zeitpunkt der ersten Alkoholbildung nach Zugabe des Katalysators zu den Reaktionspartnern deutlich verzögert (Tabelle 1).

Tabelle 1:

| Zeitpunkt der ersten Methanolabscheidung in Abhängigkeit vom Zusatzmittel | | |
|---|---|---|
| Fettsäure-N-Methylglucamid | Zusatzmittel | Methanolabscheidung in Minuten |
| $C_{12}$-$C_{24}$-Glucamid | Genagen CA 050 | 5 |
| $C_{16}$-$C_{18}$-Glucamid | Genagen CA 050 | 6 |
| $C_{16}$-$C_{18}$-Glucamid | Genagen PA 050 | 9 |
| $C_{16}$-$C_{18}$-Glucamid | Genagen OA 100 | 13 |
| $C_{12}$-$C_{24}$-Glucamid | Monopropylenglykol | 18 |
| $C_{16}$-$C_{18}$-Glucamid | Genapol LA 050 | 25 |
| $C_{16}$-$C_{18}$-Glucamid | $C_{10}$-Fettsäuredimethylamid | 31 |

Reaktionstemperatur: 120°C, bei Verwendung von $C_{10}$-Fettsäuredimethylamid:
120 - 130°C
Mischungsverhältnis: Amin/Zusatzmittel: 70/30
Katalysator: Natriummethylatlösung in Methanol

Das auf diese Weise erhaltene Produktgemisch kann ohne weitere Reinigungsmethoden in Formulierungen, beispielsweise in Wasch- und Reinigungsmittel, kosmetische Mittel, Pflanzenschutzmittel etc., eingearbeitet werden.

Die erhaltenen pastösen Mischungen verfestigen sich bei Raumtemperatur zu festen bis klebrig festen Blöken, die in einem weiteren Verfahrensschritt durch Zugabe von bei der Herstellung von Wasch- und Reinigungs- und

Geschirrspülmitteln, kosmetischen Mitteln, Pflanzenschutzmitteln üblichen Zusatzstoffen wie beispielsweise Tenside, Lösungsvermittler, Entschäumer, Builder, Trägermaterialien, Salze und Stellmittel, Bleichmittel, optische Aufheller, Vergrauungsinhibitoren und Bleichaktivatoren, Adjuvants, drift-control-Agentien, Bioziden weiterverarbeitet werden können.

Das Gewichtsverhältnis der Fettsäure-N-Alkylpolyhydroxyamide der Formel I und der Fettsäureamidoalkoxylate der Formel III kann in weiten Grenzen schwanken und beträgt im allgemeinen 90:10 bis 10:90, bevorzugt 90:10 bis 30:70, insbesondere 80:20 bis 50:50 Gew.-%.

Die nach dem erfindungsgemäßen Verfahren hergestellten Fettsäure-N-Alkylpolyhydroxyamiden der Formel I bzw. Mischungen aus Fettsäure-N-Alkylpolyhydroxyamiden der Formel I und Fettsäureamidoalkoxylate der Formel III lassen sich generell in allen Wasch- und Reinigungsmitteln jeder Art einsetzen, bevorzugt in Handgeschirrspülmitteln, flüssigen Allzweckreinigern oder flüssigen Feinwaschmitteln für die Handwäsche.

Des weiteren sind die erfindungsgemäß hergestellten Produkte geeignete nichtionische, milde Tenside, aber auch Emulgatoren für kosmetische Reinigungs-und Pflegemittel für Haut und Haare.

Darüber hinaus finden die erfindungsgemäß hergestellten Produkte Verwendung in den Bereichen Pflanzenschutz und Metallbearbeitung.

Im folgenden sind Herstellbeispiele von Fettsäure-N-Alkylpolyhydroxyamiden unter Einsatz von Fettsäureamidoalkoxylate näher beschrieben ohne die Erfindung auf diese einzuschränken.

Beispiele:

1. Herstellung von $C_{16}$-$C_{18}$-Alkyl-N-Methylglucamid in Gegenwart von Cocosfettsäureamid-ethoxylat und Natriummethylat

220,8 g (0,5 Mol) einer wässrigen Lösung aus N-Methylglucamin mit einem
M-Methylglucamin-Gehalt von 94 Gew.-% bezogen auf die Festsubstanz, wurden mit Hilfe einer Strippapparatur entwässert. Dazu wurde die Lösung auf 100°C erwärmt und zunächst bei Normaldruck bis zu einem Restwassergehalt von etwa 5 Gew.-% entwässert. Die Lösung wurde nun auf 130°C erhitzt und bei dieser Temperatur gehalten, wobei schrittweise ein Vakuum von bis zu 30 mbar angelegt wurde. Nun wurde bis zu einem Restwassergehalt von 0,2 Gew.-%, bezogen auf die erhaltene Schmelze, weiter entwässert. Zu dieser Schmelze, die 97,6 g (0,5 Mol) N-Methylglucamin enthält, wurden bei 130°C unter Rühren 102,9 g Genagen CA 050 (30 Gew.-% bezogen auf die Gesamtmischung), erwärmt auf 100°C und 150,7 g (0,54 Mol bzw. 108 Mol-% bezogen auf N-Methylglucamin) auf 100°C erwärmter Hexadecan-, Octadecansäuremethylester ($C_{16}$-Anteil 55 - 65 %,
$C_{18}$-Anteil 30-45%) zugegeben. Anschließend wurden 9,0 g bzw. 10 Mol-%, bezogen auf N-Methylglucamin, Natriummethylat (30 gew.-%ig in Methanol) innerhalb von 5 Minuten unter Rühren zugetropft. Die Temperatur der Reaktionsmischung sank auf 120°C. Die Methanolentstehung trat bereits nach ca. 6 Minuten ein. Das Abspaltungsprodukt wurde zunächst bei Normaldruck und 115°C bis 100°C, nach 10 Minuten bis 15 Minuten bei 60 mbar und 85°C bis 90°C abdestilliert. Das Vakuum wurde auf 30 mbar verbessert und bis zum Ende der Methanolbildung gehalten.

2. Herstellung von $C_{12}$-$C_{14}$-Alkyl-N-Methylglucamid in Gegenwart von Cocosfettsäuramid-ethoxylat und Natriummethylat

220,8 g (0,5 Mol) einer wässrigen Lösung aus N-Methylglucamin mit einem N-Methylglucamin-Gehalt von 94 Gew.-% bezogen auf die Festsubstanz, wurden mit Hilfe einer Strippapparatur entwässert. Dazu wurde die Lösung auf 100°C erwärmt und zunächst bei Normaldruck bis zu einem Restwassergehalt von etwa 5 Gew.-% entwässert. Die Lösung wurde nun auf 130°C erhitzt und bei dieser Temperatur gehalten, wobei schrittweise ein Vakuum von bis zu 30 mbar angelegt wurde. Nun wurde bis zu einem Restwassergehalt von 0,2 Gew.-%, bezogen auf die erhaltene Schmelze, weiter entwässert. Zu dieser Schmelze, die 97,6 g (0,5 Mol) N-Methylglucamin enthält, wurden bei 130°C unter Rühren 87,3 g Genagen CA 050 (30 Gew.-% bezogen auf die Gesamtmischung), erwärmt auf 100°C und 115,5 g (0,525 Mol bzw. 105 Mol-% bezogen auf N-Methylglucamin) auf 100°C erwärmter $C_{12}$-, $C_{14}$-Fettsäuremethylester ($C_{12}$-Anteil 65 - 75 %, $C_{14}$-Anteil 20 - 30%, $C_{16}$-Anteil 4 - 8 %) zugegeben. Anschließend wurden 9,0 g bzw. 10 Mol-%, bezogen auf N-Methylglucamin, Natriummethylat (30 gew.-%ig in Methanol) innerhalb von 5 Minuten unter Rühren zugetropft. Die Temperatur der Reaktionsmischung sank auf 120°C. Die Methanolentstehung trat bereits nach ca. 5 Minuten ein. Das Abspaltungsprodukt wurde zunächst bei Normaldruck und 115°C bis 100°C, nach 10 Minuten bis 15 Minuten bei 60 mbar und 85°C bis 90°C abdestilliert. Das Vakuum wurde auf 30 mbar vermindert und bis zum Ende der Methanolbildung gehalten.

3. Herstellung von $C_{16}$-$C_{18}$-Alkyl-N-Methylglucamid in Gegenwart von Palmölfettsäureamid-ethoxylat und Natriummethylat

220,8 g (0,5 Mol) einer wässrigen Lösung aus N-Methylglucamin mit einem N-Methylglucamin-Gehalt von 94 Gew.-% bezogen auf die Festsubstanz, wurden mit Hilfe einer Strippapparatur entwässert. Dazu wurde die Lösung auf 100°C erwärmt und zunächst bei Normaldruck bis zu einem Restwassergehalt von etwa 5 Gew.-% entwässert. Die Lösung wurde nun auf 130°C erhitzt und bei dieser Temperatur gehalten, wobei schrittweise ein Vakuum von bis zu 30 mbar angelegt wurde. Nun wurde bis zu einem Restwassergehalt von 0,2 Gew.-%, bezogen auf die erhaltene Schmelze weiter entwässert. Zu dieser Schmelze, die 97,6 g (0,5 Mol) N-Methylglucamin enthält, wurden bei 130°C unter Rühren 102,9 g Palmölfettsäureamid-ethoxylat (30 Gew.-% bezogen auf die Gesamtmischung), erwärmt auf 100°C und 150,7 g (0,54 Mol bzw. 108 Mol-% bezogen auf N-Methylglucamin) auf 100°C erwärmtes Hexadecan-, Octadecansäuremethylester ($C_{16}$-Anteil 55 - 65 %, $C_{18}$-Anteil 30-45%) zugegeben. Anschließend wurden 9,0 g bzw. 10 Mol-%, bezogen auf N-Methylglucamin, Natriummethylat (30 gew.-%ig in Methanol) innerhalb von 5 Minuten unter Rühren zugetropft. Die Temperatur der Reaktionsmischung sank auf 120°C. Die Methanolentstehung trat bereits nach ca. 9 Minuten ein. Das Abspaltungsprodukt wurde zunächst bei Normaldruck und 115°C bis 100°C, nach 10 Minuten bis 15 Minuten bei 60 mbar und 85°C bis 90°C abdestilliert. Das Vakuum wurde auf 30mbar vermindert und bis zum Ende der Methanolbildung gehalten.

4. Herstellung von $C_{16}$-$C_{18}$-Alkyl-N-Methylglucamid in Gegenwart von Ölsäureamidethoxylat und Natriummethylat

220,8 g (0,5 Mol) einer wässrigen Lösung aus N-Methylglucamin mit einem N-Methylglucamin-Gehalt von 94 Gew.-% bezogen auf die Festsubstanz, wurden mit Hilfe einer Strippapparatur entwässert. Dazu wurde die Lösung auf 100°C erwärmt und zunächst bei Normaldruck bis zu einem Restwassergehalt von etwa 5 Gew.-% entwässert. Die Lösung wurde nun auf 130°C erhitzt und bei dieser Temperatur gehalten, wobei schrittweise ein Vakuum von bis zu 30 mbar angelegt wurde. Nun wurde bis zu einem Restwassergehalt von 0,2 Gew.-%, bezogen auf die erhaltene Schmelze, weiter entwässert. Zu dieser Schmelze, die 97,6 g (0,5 Mol) N-Methylglucamin enthält, wurden bei 130°C unter Rühren 102,9 g Ölsäureamidethoxylat (30 Gew.-% bezogen auf die Gesamtmischung), erwärmt auf 100°C und 150,7 g (0,54 Mol bzw. 108 Mol-% bezogen auf N-Methylglucamin) auf 100°C erwärmtes Hexadecan-, Octadecansäuremethylester ($C_{16}$-Anteil 55 - 65 %, $C_{18}$-Anteil 30 - 45%) zugegeben. Anschließend wurden 9,0 g bzw. 10 Mol-%, bezogen auf N-Methylglucamin, Natriummethylat (30 gew.-%ig in Methanol) innerhalb von 5 Minuten unter Rühren zugetropft. Die Temperatur der Reaktionsmischung sank auf 120°C. Die Methanolentstehung trat bereits nach ca. 13 Minuten ein. Das Abspaltungsprodukt wurde zunächst bei Normaldruck und 115°C bis 100°C, nach 10 Minuten bis 15 Minuten bei 60 mbar und 85°C bis 90°C abdestilliert. Das Vakuum wurde auf 30 mbar vermindert und bis zum Ende der Methanolbildung gehalten.

5. Herstellung von $C_{16}$-$C_{18}$-Alkyl-N-Methylglucamid in Gegenwart von Cocosfettsäureamid-ethoxylat und NaOH

220,8 g (0,5 Mol) einer wässrigen Lösung aus N-Methylglucamin mit einem N-Methylglucamin-Gehalt von 94 Gew.-% bezogen auf die Festsubstanz und 4,0 g 50 gew.-%ige Natronlauge (10 mol-% bezogen auf N-Methylglucamin), wurden mit Hilfe einer Strippapparatur entwässert. Dazu wurde die Lösung auf 100°C erwärmt und zunächst bei Normaldruck bis zu einem Restwassergehalt von etwa 5 Gew.-% entwässert. Die Lösung wurde nun auf 130°C erhitzt und bei dieser Temperatur gehalten, wobei schrittweise ein Vakuum von bis zu 30 mbar angelegt wurde. Nun wurde bis zu einem Restwassergehalt von 0,2 Gew.-%, bezogen auf die erhaltene Schmelze, weiter entwässert. Zu dieser Schmelze, die 97,6 g (0,5 Mol) N-Methylglucamin enthält, wurden bei 130°C unter Rühren 102,9 g Genagen CA 050 (30 Gew.-% bezogen auf die Gesamtmischung), erwärmt auf 100°C und anschließend 150,7 g (0,54 Mol bzw. 108 Mol-% bezogen auf N-Methylglucamin) auf 100°C erwärmtes Hexadecan-, Octadecansäuremethylester ($C_{16}$-Anteil 55 - 65 %, $C_{18}$-Anteil 30 - 45%) innerhalb von ca. 15 Minuten zugetropft. Die Temperatur der Reaktionsmischung sank auf 120°C. Die Methanolentstehung trat bereits nach ca. 5 Minuten ein. Das Abspaltungsprodukt wurde zunächst bei Normaldruck und 115 °C bis 100°C, nach 10 Minuten bis 15 Minuten bei 60 mbar und 85°C bis 90°C abdestilliert. Das Vakuum wurde auf 30mbar vermindert und bis zum Ende der Methanolbildung gehalten.

6. Herstellung von $C_{16}$-$C_{18}$-Alkyl-N-Methylglucamid in Gegenwart von Laurylalkohol-ethoxylat und Natriummethylat

220,8 g (0,5 Mol) einer wässrigen Lösung aus N-Methylglucamin mit einem N-Methylglucamin-Gehalt von 94 Gew.-% bezogen auf die Festsubstanz, wurden mit Hilfe einer Strippapparatur entwässert. Dazu wurde die Lösung auf 100°C erwärmt und zunächst bei Normaldruck bis zu einem Restwassergehalt von etwa 5 Gew.-% entwässert. Die Lösung wurde nun auf 130°C erhitzt und bei dieser Temperatur gehalten, wobei schrittweise ein Vakuum von bis zu 30 mbar angelegt wurde. Nun wurde bis zu einem Restwassergehalt von 0,2 Gew.-%, bezogen auf die erhaltene

Schmelze, weiter entwässert. Zu dieser Schmelze, die 97,6 g (0,5 Mol) N-Methylglucamin enthält, wurden bei 130°C unter Rühren 102,9 g Genapol LA 050 (30 Gew.-% bezogen auf die Gesamtmischung), erwärmt auf 100°C und 150,7 g (0,54 Mol bzw. 108 Mol-% bezogen auf N-Methylglucamin) auf 100°C erwärmter Hexadecan-, Octadecansäuremethylester ($C_{16}$-Anteil 55 - 65 %, $C_{18}$-Anteil 30 - 45 %) zugegeben. Anschließend wurden 9,0 g bzw. 10 Mol-%, bezogen auf N-Methylglucamin, Natriummethylat (30 gew.-%ig in Methanol) innerhalb von 5 Minuten unter Rühren zugetropft. Die Temperatur der Reaktionsmischung sank auf 120°C. Die Methanolentstehung trat nach ca. 25 Minuten ein. Das Abspaltungsprodukt wurde zunächst bei Normaldruck und 115°C bis 100°C, nach 10 Minuten bis 15 Minuten bei 60 mbar und 85°C bis 90°C abdestilliert. Das Vakuum wurde auf 30mbar vermindert und bis zum Ende der Methanolbildung gehalten.

7. Herstellung von $C_{12}$-$C_{14}$-Alkyl-N-Methylglucamid in Gegenwart von oxethyliertem Cocosfettsäureethanolamid und Natriummethylat

220,8 g (0,5 Mol) einer wässrigen Lösung aus N-Methylglucamin mit einem N-Methylglucamin-Gehalt von 94 Gew.-% bezogen auf die Festsubstanz, wurden mit Hilfe einer Strippapparatur entwässert. Dazu wurde die Lösung auf 100°C erwärmt und zunächst bei Normaldruck bis zu einem Restwassergehalt von etwa 5 Gew.-% entwässert. Die Lösung wurde nun auf 130°C erhitzt und bei dieser Temperatur gehalten, wobei schrittweise ein Vakuum von bis zu 30 mbar angelegt wurde. Nun wurde bis zu einem Restwassergehalt von 0,2 Gew.-%. bezogen auf die erhaltene Schmelze, weiter entwässert. Zu dieser Schmelze, die 97,6 g (0,5 Mol) N-Methylglucamin enthält, wurden bei 130°C unter Rühren 87,3 g Genagen CA 050 (30 Gew.-% bezogen auf die Gesamtmischung), erwärmt auf 100°C und 115,5 g (0,525 Mol bzw. 105 Mol-% bezogen auf N-Methylglucamin) auf 100°C erwärmter $C_{12}$-, $C_{14}$-Fettsäuremethylester ($C_{12}$-Anteil 65 - 75 %, $C_{14}$-Anteil 20 - 30 %, $C_{16}$-Anteil 4 - 8 %) zugegeben. Anschließend wurden 9,0 g bzw. 10 Mol-%, bezogen auf N-Methylglucamin, Natriummethylat (30 gew.-%ig in Methanol) innerhalb von 5 Minuten unter Rühren zugetropft. Die Temperatur der Reaktionsmischung sank auf 120°C. Die Methanolentstehung trat nach ca. 18 Minuten ein. Das Abspaltungsprodukt wurde zunächst bei Normaldruck und 115°C bis 100°C, nach 10 Minuten bis 15 Minuten bei 60 mbar und 85°C bis 90°C abdestilliert. Das Vakuum wurde auf 30 mbar vermindert und bis zum Ende der Methanolbildung gehalten.

| Chemische Bezeichnung der eingesetzten Handelsprodukte: | |
|---|---|
| Genagen CA 050 | Cocosfettsäuremonoethanolamid mit 5 EO |
| Genagen PA 050 | Palmölmonoethanolamid mit 5 EO |
| Genagen OA 100 | Ölsäuremonoethanolamid mit 10 EO |
| Genapol LA 050 | $C_{12}$-$C_{16}$-Alkoholethoxylat mit 5 EO |

Die oben genannten Produkte sind Handelsprodukte der Clariant GmbH, Frankfurt am Main

**Patentansprüche**

1. Verfahren zur Herstellung von Fettsäure-N-alkylpolyhydroxyamiden der Formel

$$R^1CONR^2Z$$

worin $R^1$ für eine lineare oder verzweigte, gesättigte oder ungesättigte Alkylgruppe mit 7 bis 21 Kohlenstoffatomen, Z für eine Polyhydroxykohlenwasserstoffgruppe mit mindestens drei Hydroxylgruppen, die auch alkoxyliert sein können, und $R^2$ für H, $C_1$-$C_8$-Alkyl, eine Gruppe der Formeln $-(CH_2)_xNR^3R^4$ oder $R^5O(CH_2)_n-$, $R^3$ und $R^4$ für $C_1$-$C_4$-Alkyl oder $C_2$-$C_4$-Hydroxyalkyl, $R^5$ für $C_1$-$C_4$-Alkyl, n für eine Zahl von 2 bis 4 und x für eine Zahl von 2 bis10, stehen, durch Umsetzung eines N-Alkyl-polyhydroxyamins der Formel

$$H\text{-}NR^2Z$$

mit einer Fettsäure, Fettsäurechlorid oder Fettsäureesters in Gegenwart eines basischen Katalysators, dadurch gekennzeichnet, dass man die Reaktion in Gegenwart eines Fettsäureamidoalkoxylats der Formel

$$R\text{-}CONR^6R^7$$

durchführt, wobei R Alkyl oder Alkenyl mit 7 bis 21 Kohlenstoffatomen, $R^6$ Wasserstoff oder eine Gruppe $-(AO)_xH$, $R^7$ eine Gruppe $-(AO)_xH$, A eine Gruppe der Formeln $-C_2H_4-$, $-C_3H_6-$ oder $-C_4H_8-$ und x eine Zahl von 1 bis 20 bedeuten.

**2.** Verfahren nach Anspruch 1, dadurch gekennzeichnet, dass man Fettsäure-N-alkylglucamide der Formel

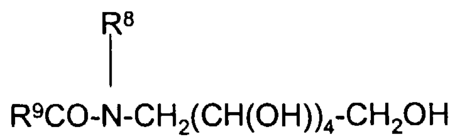

herstellt, wobei $R^8$ $C_1$-$C_4$-Alkyl und $R^9CO$ den Rest einer gesättigten oder ungesättigten Fettsäure mit 12 bis 18 C-Atomen bedeuten.

**3.** Verfahren nach Anspruch 1, dadurch gekennzeichnet, dass man die Reaktion in Gegenwart eines Metallalkoxids, eines Alkalihydroxids oder eines Alkalisalzes von Glycerin oder Propylenglykol durchführt.

**4.** Verwendung des Produktgemisches, erhalten nach Anspruch 1, als Tensid oder Emulgator in Wasch- und Reinigungsmitteln sowie in kosmetischen Zubereitungen, in Pflanzenschutzformulierungen und in Zubereitungen für die Metallbearbeitung.